# EUROPÄISCHE PATENTSCHRIFT

(11) **EP 1 675 517 B1**
(45) Veröffentlichungstag und Bekanntmachung des Hinweises auf die Patenterteilung: **05.08.2009**
(21) Anmeldenummer: 03753201.7
(22) Anmeldetag: 24.10.2003
(51) Int. Cl.: A61B 17/80, A61B 17/86

(54) **VORRICHTUNG ZUR KNOCHENFIXATION**
BONE FIXATION DEVICE
DISPOSITIF DE FIXATION OSSEUSE

(43) Veröffentlichungstag der Anmeldung: 05.07.2006
(73) Patentinhaber: Synthes GmbH, 4436 Oberdorf (CH)
(72) Erfinder: MATTHYS, Romano, CH-7272 Davos-Clavadel (CH)
(74) Vertreter: Lusuardi, Werther
(86) Internationale Anmeldenummer: PCT/CH2003/000697
(87) Internationale Veröffentlichungsnummer: WO 2005/039426

(56) Entgegenhaltungen:
- WO-A-00/66011
- DE-A- 4 343 117
- DE-A- 19 629 011
- US-A1- 2002 045 897
- US-A1- 2003 153 919

## Beschreibung

Die Erfindung bezieht sich auf eine Vorrichtung zur Knochenfixation gemäss dem Oberbegriff des Patentanspruchs 1.

Solche Vorrichtungen eignen sich insbesondere für die Versorgung von Knochenfrakturen in gelenknahen Bereichen, z.B. am proximalen Humerus.

Eine Vorrichtung mit einer mehrere Bohrungen umfassenden Knochenplatte und mehreren Schrauben, deren Aussengewindedurchmesser grösser als der Durchmesser der Plattenbohrungen ist, ist aus WO 00/66011 MICHELSON Basis für der Oberbegriff des Anspruchs 1 bekannt.

Aus der WO01/12081 ist eine gattungsgemässe Vorrichtung bekannte. Allerdings sind die in den Plattenbohrungen fakultativ vorgesehenen Innengewinde gleich dimensioniert wie die Aussengewinde der longitudinalen Knochenfixationsmittel, so dass sich letztere nicht in das Material der Knochenplatte einschneiden können und dadurch weder eine axiale noch eine rotative Stabilität erreicht wird.

Aus der WO01/93768 ist eine Knochenplatte bekannt, welche aus einem bioresorbierbaren Kunststoff besteht, jedoch lediglich Vertiefungen in der Platte aufweist, statt durchgehender Bohrungen. Der Nachteil dieser bekannten Platte besteht somit darin, dass die in die Vertiefungen eingebrachten longitudinalen Knochenfixationsmittel vollständig durch die restliche Plattendicke hindurchgebohrt werden müssen. Einerseits sind dazu spezielle longitudinale Knochenfixationsmittel mit einer Bohrerspitze notwendig und anderseits entstehen durch diesen Vorgang eine grössere Menge an Spänen, welche zumindest während einer gewissen Zeit im Körper verbleiben.

Hier will die Erfindung Abhilfe schaffen. Der Erfindung liegt die Aufgabe zugrunde, eine Vorrichtung zur Knochenfixation zu schaffen, welche in der Lage ist zwei oder mehrere longitudinale Knochenfixationselemente miteinander über einen Knochenplatte, bestehend aus einem polymeren Kunststoff, winkelstabil und rotationsgesichert, zu verbinden.

Die Erfindung löst die gestellte Aufgabe mit einer Vorrichtung, welche die Merkmale des Anspruchs 1 aufweist.

Die durch die Erfindung erreichten Vorteile sind im wesentlichen darin zu sehen, dass das Verankerungsgewinde während des Implantierens der Knochenfixationselemente in die Knochenplatte, bestehend aus einem polymeren Kunststoff, geformt wird. Während dieses Umform-Vorganges entsteht eine unbedenkliche Menge an Abriebmaterial, jedoch keine eigentlichen Späne. Durch das Umformen des Verankerungsgewindes während des Eindrehens des longitudinalen Knochenfixationselementes in die Knochenplatte, wird dieses winkelstabil und rotationsgesichert mit der Knochenplatte verbunden. Diese Verbindungstechnik gibt dem Anwender einen bestimmten Spielraum bezüglich der Positionierungsgenauigkeit des longitudinalen Knochenfixationselementes gegenüber der Bohrung in der Knochenplatte.

Die Knochenplatte der erfindungsgemässen Vorrichtung weist Plattenlöcher auf, welche einen kleineren Durchmesser aufweisen, als der Durchmesser des Aussengewindes der in die Plattenlöcher einzuführenden longitudinalen Knochenfixationsmittel, jedoch grösser sind als der Kerndurchmesser dieses Aussengewindes. Durch diese Durchmesserdifferenzen wird in den Plattenlöchern der Knochenplatte in situ beim Eindrehen des longitudinalen Knochenfixationsmittels ein entsprechendes Gewinde geformt. Die wirkende, radiale Vorspannung am Aussengewinde des longitudinalen Knochenfixationsmittels in der Kontaktzone (Plattenloch) der Knochenplatte, resultierend aus dem Gewindeumformen, sichert das longitudinale Knochenfixationsmittel ausreichend auf darauf einwirkende Rotationsmomente. Dies verhindert wiederum eine Längsverschiebung des longitudinalen Knochenfixationsmittels relativ zur Knochenplatte.

Bei einer besonderen Ausführungsform besteht die Knochenplatte aus einem im wesentlichen nicht-resorbierbaren Kunststoff, vorzugsweise aus PEEK. Dadurch erhält man überraschenderweise eine besonders gute Fixation der Knochenplatte.

Die Knochenplatte kann zusätzlich eine zentrale Bohrung zur Aufnahme eines Zielgerätes umfassen.

Bei einer weiteren Ausführungsform weisen die Lochachsen eines oder mehrerer der N Plattenlöcher einen Winkel alpha > 0° zur Normalen auf der Knochenplatte auf.

Der Betrag "D" für den Durchmesser der Plattenlöcher beträgt vorzugsweise mindestens das 1,05-fache von "K" und höchstens das 1,15-fache von "K" ("K" = Kerndurchmesser des Knochenfixationselementes).

Bei einer weiteren Ausführungsform verlaufen die Lochachsen von mindestens zwei, vorzugsweise von mindestens drei der N Plattenlöcher windschief zueinander. Dadurch ergibt sich eine verbesserte Verankerung der gesamten Vorrichtung im Knochen.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht der erfindungsgemässen Vorrichtung;
Fig. 2 eine Aufsicht auf die Knochenplatte der Fig. 1;
Fig. 3 einen Längsschnitt längs der Linie III-III in Fig. 2;
Fig. 4 einen Längsschnitt längs der Linie IV-IV in Fig. 2; und
Fig. 5 einen vergrösserten Teilschnitt aus Fig. 3.

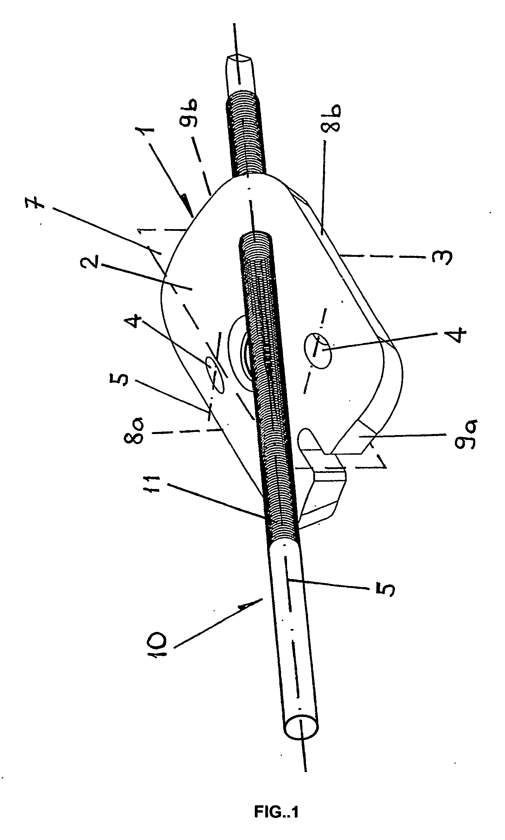

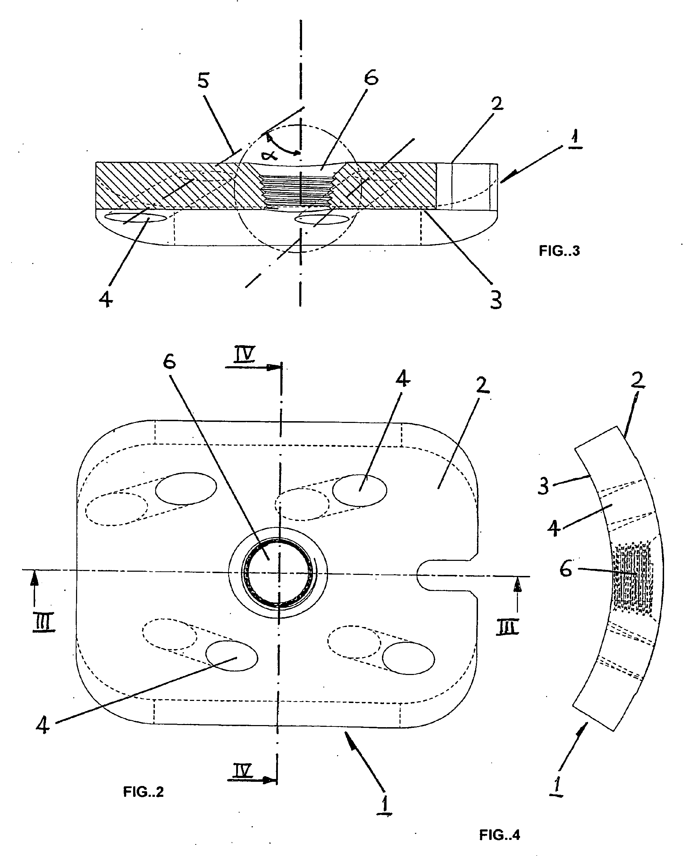

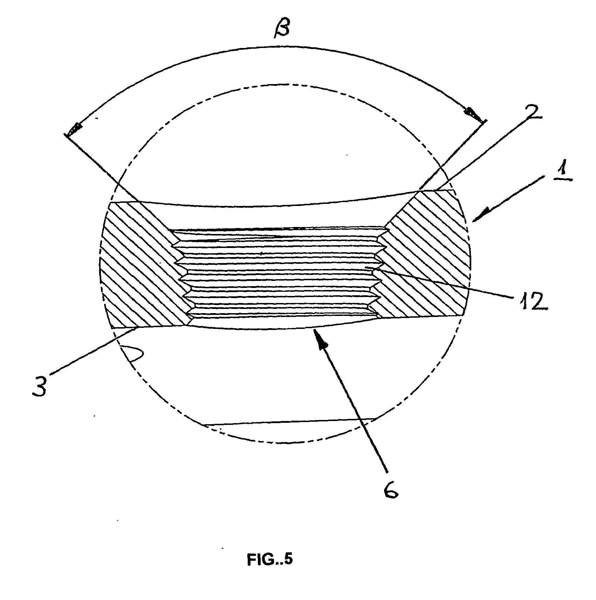

In den Fig. 1 bis 5 ist eine Ausführungsform dargestellt, welche eine konvex gewölbte Knochenplatte 1 und ein in eines der Plattenlöcher 4 eingeschraubtes Knochenfixationselement 10 umfasst. Die Knochenplatte 1 ist oval mit abgerundeten Ecken ausgebildet und in orthogonal zur Mittelebene 7 stehenden Querschnittsflächen konvex gekrümmt. Die Lochachsen 5 der vier Plattenlöcher 4 stehen windschief relativ zueinander und schräg zur Oberseite 2 der Knochenplatte 1. Die Knochenplatte 1 weist in der hier dargestellten Ausführungsform zwei lange Seitenflächen 8a;8b und zwei kurze Seitenflächen 9a;9b sowie eine die zwei kurzen Seitenflächen 9a;9b schneidende Mittelebene 7 auf. Bezüglich der Mittelebene 7 symmetrisch ausgestaltet ist lediglich der Körper der Knochenplatte 1, während die Anordnung der Plattenlöcher 4 zur Mittelebene 7 nicht symmetrisch ist. Die Abstände der Lochachsen 5 der Plattenlöcher 4 zur Mittelebene 7 sowie zu den kurzen Seitenflächen 9a;9b der Knochenplatte 1 sind für die vier Plattenlöcher 4 verschieden. Ferner weisen die Lochachsen 5 der vier Plattenlöcher 4 zur Normalen auf der Knochenplatte 1 einen Winkel α zwischen 20° und 80° auf.

Die Plattenlöcher 4 durchdringen die Knochenplatte 1 derart, dass die Schnittpunkte der Lochachsen 5 mit der Oberseite 2 näher bei der zweiten kurzen Seitenfläche 9b liegen als die Schnittpunkte mit der Unterseite 3. Die Knochenfixationselemente 10 können somit alle von derselben Seite der Knochenplatte 1 her in diese eingeschraubt werden.

Die Plattenlöcher 4 weisen einen kleineren Durchmesser auf als der Aussendurchmesser des Aussengewindes 11 der Knochenfixationsmittel 10. Da zudem der Kerndurchmesser des Aussengewindes 11 kleiner ist als der Durchmesser der Plattenlöcher 4 werden die Knochenfixationsmittel 10 beim Einschrauben in die Knochenplatte 10 durch Umformung des Wandmaterials in der Bohrung an dieser fixiert. Die Knochenplatte 1 ist aus einem polymeren Material gefertigt, so dass die Aussengewinde 11 der Knochenfixationselemente 10 einfacher in die Knochenplatte 1 einschraubbar sind.

Zur Befestigung eines Zielgerätes (nicht gezeichnet) umfasst die Knochenplatte 1 eine zentrale Bohrung 6, welche die Knochenplatte 1 mittig von der Oberseite 2 bis zur Unterseite 3 durchdringt. Die zentrale Bohrung 6 ist konisch ausgebildet und auf dem an die Unterseite 3 grenzenden Längenabschnitt x mit einem konischen Innengewinde 12 versehen. Der an die Oberseite 2 grenzende, gewindefreie Längenabschnitt y der zentralen Bohrung 6 ist mit einem grösseren Konuswinkel β ausgestaltet. In der hier dargestellten Ausführungsform ist x > y.

## Patentansprüche

1. Vorrichtung zur Knochenfixation umfassend
A) eine aus einem polymeren Material gefertigte Knochenplatte (1) mit einer Oberseite (2), einer zur Anlage an den Knochen bestimmten Unterseite (3) und N ≥ 2 die Oberseite (2) mit der Unterseite (3) verbindende Plattenlöcher (4) des Durchmessers "D" und mit einer Lochachse (5); und
B) mehrere, zur Einführung in die N Plattenlöcher (4) bestimmte, longitudinale Knochenfixationselemente (10) mit einem Aussengewinde (11) vom Durchmesser "A" und einem Kerndurchmesser "K", wobei
C) die Beziehung K < D < A gilt, so dass
D) durch das Aussengewinde (11) beim Eindrehen des Knochenfixationselementes (10) in situ ein entsprechendes Gewinde im Plattenloch (4) der Knochenplatte (1) geformt wird
**dadurch gekennzeichnet, dass**
E) A mindestens das 1,2-fache und höchstens das 1,3-fache von K beträgt.

2. Vorrichtung nach Anspruch 1, **dadurch gekennzeichnet, dass** die Knochenplatte (1) aus einem im wesentlichen nicht-resorbierbaren Kunststoff, vorzugsweise aus PEEK besteht.

3. Vorrichtung nach Anspruch 1 oder 2, **dadurch gekennzeichnet, dass** sie zusätzlich eine zentrale Bohrung (6) zur Aufnahme eines Zielgerätes umfasst.

4. Vorrichtung nach einem der Ansprüche 1 bis 3, **dadurch gekennzeichnet, dass** die Lochachsen (5) eines oder mehrere der N Plattenlöcher (4) einen Winkel alpha > 0° zur Normalen auf der Knochenplatte (1) aufweisen.

5. Vorrichtung nach einem der Ansprüche 1 bis 4, **dadurch gekennzeichnet, dass** D mindestens das 1,05-fache von K beträgt.

6. Vorrichtung nach einem der Ansprüche 1 bis 4, **dadurch gekennzeichnet, dass** D höchstens das 1,15-fache von K beträgt.

7. Vorrichtung nach einem der Ansprüche 1 bis 6, **dadurch gekennzeichnet, dass** die Lochachsen (5) von mindestens zwei, vorzugsweise von mindestens drei der N Plattenlöcher (4) windschief zueinander verlaufen.

## Claims

1. Device for bone fixation, comprising:
A) a bone plate (1) made of a polymeric material, with an upper side (2), a lower side (3) destined for contacting the bone and N ≥ 2 plate holes (4) connecting the upper side (2) with the lower side (3), having a diameter "D" and a hole axis (5); and
B) several longitudinal bone fixation elements (10) destined for insertion into the N plate holes (4), having an outer thread (11) with a diameter "A" and a core diameter "K", where
C) the relationship K < D < A applies, so that
D) due to the outer thread (11), the motion of screwing in the bone fixation element (10) in-situ forms a corresponding thread in the plate hole (4) of the bone plate (1),
**characterized in that**
E) A is at least 1,2 times and at the most 1,3 times the size of K.

2. Device according to claim 1, **characterized in that** the bone plate (1) consists of an essentially non-resorbable synthetic material, preferably PEEK.

3. Device according to claim 1 or 2, **characterized in that** it additionally comprises a central borehole (6) to receive an aiming device.

4. Device according to one of the claims from 1 to 3, **characterized in that** the hole axes (5) of one or more of the N plate holes (4) form an angle alpha > 0° with the perpendicular to the bone plate (1).

5. Device according to one of the claims from 1 to 4, **characterized in that** D is at least 1,05 times the size of K.

6. Device according to one of the claims from 1 to 4, **characterized in that** D is at the most 1,15 times the size of K.

7. Device according to one of the claims from 1 to 6, **characterized in that** the hole axes (5) of at least two, preferably of at least three of the N plate holes (4) run in a direction skewed to each other.

## Revendications

1. Dispositif de fixation osseuse comprenant :
A/ une plaque osseuse (1) fabriquée dans un matériau polymère, comportant un dessus (2), un dessous (3) destiné à venir en appui sur l'os et un nombre - supérieur ou égal à 2 - de perçages de plaque (4), du diamètre "D", reliant le dessus (2) et le dessous (3) et comportant un axe de perçage (5) ; et
B/plusieurs éléments de fixation osseuse (10), longitudinaux, servant à l'introduction dans les N perçages de plaque (4), lesdits éléments ayant un filetage extérieur (11) du diamètre "A" et un diamètre du noyau "K", où
C/ la relation K < D < A s'applique, de sorte que,
D/sous l'effet du filetage extérieur (11), en vissant l'élément de fixation osseuse (10), in situ, un filetage correspondant est formé dans le perçage de plaque (4) de la plaque osseuse (1),
**caractérisé en ce que**
E/ le diamètre A est égal au moins à 1,2 fois et au plus à 1,3 fois le diamètre K.

2. Dispositif selon la revendication 1, **caractérisé en ce que** la plaque osseuse (1) est réalisée dans une matière plastique sensiblement non résorbable, de préférence en utilisant du polyétheréthercétone (PEEK).

3. Dispositif selon la revendication 1 ou 2, **caractérisé en ce qu'**il comporte en outre un perçage central (6) servant au logement d'un appareil de visée.

4. Dispositif selon l'une des revendications 1 à 3, **caractérisé en ce que** les axes de perçage (5) d'un ou de plusieurs N trous de plaque (4) présentent un angle alpha > 0° par rapport à la normale sur la plaque osseuse (1).

5. Dispositif selon l'une des revendications 1 à 4, **caractérisé en ce que** le diamètre D est égal au moins à 1,05 fois le diamètre K.

6. Dispositif selon l'une des revendications 1 à 4, **caractérisé en ce que** le diamètre D est égal au plus à 1,15 fois le diamètre K.

7. Dispositif selon l'une des revendications 1 à 6, **caractérisé en ce que** les axes de perçage (5) d'au moins deux, de préférence d'au moins trois des N perçages de plaque (4), s'étendent de façon oblique les uns par rapport aux autres.
